# EUROPEAN PATENT APPLICATION

(11) **EP 1 975 244 A1**
(43) Date of publication of application: **01.10.2008**
(21) Application number: 07706273.5
(22) Date of filing: 19.01.2007
(51) Int. Cl.: C12Q 1/44, C12Q 1/26, C07K 16/18

(54) **METHOD FOR MEASURING CHOLESTEROL IN REMNANT-LIKE LIPOPROTEIN**

(30) Priority: 20.01.2006 JP 2006013012
(71) Applicant: Jimro Co., Ltd., Takasaki-shi, Gumma 370-0021 (JP)
(72) Inventor: OGIHARA, Fumiko, Fujioka-shi, Gunma 375-0024 (JP); NIIMI, Manabu, Takasaki-shi, Gunma 370-0046 (JP); NAKANO, Takamitsu, Maebashi-shi, Gunma 371-0846 (JP); KANATANI, Kazushi, Isesaki-shi, Gunma 370-0128 (JP)
(74) Representative: Hartz, Nikolai
(86) International application number: PCT/JP2007/000021
(87) International publication number: WO 2007/083523

(57) **Abstract**

A method for measuring cholesterol in remnant-like lipoprotein in a sample, by a more convenient operation and with high sensitivity, and a reagent for the measurement are provided.

A method for measuring cholesterol in remnant-like lipoprotein, the method comprising subjecting a test sample containing lipoproteins to the action of a cholesterol esterase and a cholesterol oxidase or cholesterol dehydrogenase, and measuring the hydrogen peroxide or reduced coenzyme generated by the enzymatic reaction, wherein a surfactant having a benzenesulfonic acid structure in the molecule or a polyacrylic acid-based polymeric surfactant is used.

## Description

### Technical Field

The present invention relates to a method and a reagent for measuring cholesterol in remnant-like lipoprotein which is considered as a risk factor of arteriosclerosis and the like in clinical examinations.

### Background Art

In clinical examinations, cholesterol in high density lipoprotein (HDL) is considered as a negative risk factor of arteriosclerosis, while cholesterol in low density lipoprotein (LDL) is considered as a positive risk factor of arteriosclerosis. Thus, these cholesterols are frequency measured in the field of clinical examinations.

In recent years, it has been reported that cholesterol in a lipoprotein which is generated as a result of lipid metabolism or the like, such as remnant-like lipoprotein, may provide a more closely related indicator than the cholesterol in LDL, as a risk factor of arteriosclerosis. Thus, it is a problem at issue to efficiently measure the cholesterol in remnant-like lipoprotein among the lipoproteins in a biological sample.

Among the methods for enzymatically measuring cholesterol in remnant-like lipoprotein, a method of conducting measurement by adding an enzyme for degrading phospholipids to cholesterol esterase and cholesterol oxidase or cholesterol dehydrogenase, and optionally, further adding a surfactant, has been reported (see, for example, Patent Document 1).
However, even such method cannot be necessarily said to be sufficient in terms of the selectivity of enzymatic reaction or convenience.
[Patent Document 1] JP-A-2001-231597

### Disclosure of the Invention

### Problems to be Solved by the Invention

It is an object of the present invention to provide a method and a reagent for measuring cholesterol in remnant-like lipoprotein in a sample, by a more convenient operation and with good sensitivity.

The inventors of the present invention investigated, under such circumstances, various conditions for measuring cholesterol in lipoprotein using a cholesterol esterase and a cholesterol oxidase or cholesterol dehydrogenase, and as a result, found that cholesterol in remnant-like lipoprotein in a biological sample containing lipoproteins such as high density lipoprotein (HDL), low density lipoprotein (LDL), chylomicron (CM) , very low density lipoprotein (VLDL), CM remnants, VLDL remnants and intermediate density lipoprotein (IDL) remnants, can be measured selectively with high sensitivity using a specific surfactant, thus completing the present invention.

### Means for solving the Problems

Thus, the present invention is directed to a method for measuring cholesterol in remnant-like lipoprotein, the method comprising subjecting a test sample containing lipoproteins to the action of a cholesterol esterase and a cholesterol oxidase or cholesterol dehydrogenase, and measuring the hydrogen peroxide or reduced coenzyme generated by the enzymatic reaction, wherein a surfactant having a benzenesulfonic acid structure in the molecule or a polyacrylic acid-based polymeric surfactant is used.

The present invention is also directed to a reagent for measuring cholesterol in remnant-like lipoprotein, the reagent comprising a cholesterol esterase and a cholesterol oxidase or cholesterol dehydrogenase, and a surfactant having a benzenesulfonic acid structure in the molecule or a polyacrylic acid-based polymeric surfactant.

### Effects of the Invention

When the measurement method and the reagent for measurement of the present invention are used, cholesterol in remnant-like lipoprotein can be measured in a very convenient manner with good sensitivity.

### Brief Description of the Drawings

Fig. 1 is a graph showing the respective reaction curves for lipoproteins in the case of using invented product 1 of the present invention.
Fig. 2 is a graph showing the respective reaction curves for lipoproteins in the case of using invented product 2 of the present invention.
Fig. 3 is a graph showing the respective reaction curves for lipoproteins in the case of using invented product 3 of the present invention.
Fig. 4 is a graph showing the respective reaction curves for lipoproteins in the case of using invented product 4 of the present invention.
Fig. 5 is a graph showing the respective reaction curves for lipoproteins in the case of using invented product 5 of the present invention.
Fig. 6 is a graph showing the respective reaction curves for lipoproteins in the case of using invented product 6 of the present invention.
Fig. 7 is a graph showing the respective reaction curves for lipoproteins in the case of using comparative product 1.
Fig. 8 is a graph showing the respective reaction curves for lipoproteins in the case of using comparative product 2.
Fig. 9 is a graph showing the respective reaction curves for lipoproteins in the case of using comparative product 3.
Fig. 10 is a graph showing the respective reaction curves for lipoproteins in the case of using comparative product 4.
Fig. 11 is a graph showing the respective reaction curves for lipoproteins in the case of using comparative product 5.
Fig. 12 is a graph showing the respective reaction curves for lipoproteins in the case of using comparative product 6.
Fig. 13 is a graph showing the respective reaction curves for lipoproteins in the case of using comparative product 7.
Fig. 14 is a graph showing the respective reaction curves for lipoproteins in the case of using comparative product 8.
Fig. 15 is a graph showing the correlation between the absorbance values determined by the measurement method of the present invention (ordinate axis) , and the values obtained using a commercially available RLP-cholesterol measurement kit (abscissa axis) .
Fig. 16 is a graph showing the correlation between the absorbance values determined by the measurement method of the present invention (ordinate axis) , and the values obtained using a commercially available RLP-cholesterol measurement kit (abscissa axis).
Fig. 17 is a graph showing the correlation between the absorbance values determined by the measurement method of the present invention (ordinate axis), and the values obtained using a commercially available RLP-cholesterol measurement kit (abscissa axis) ((A): anti-apoB-100 antibody not added, (B): anti-apoB-100 antibody added).

### Best Mode for Carrying Out the Invention

The measurement method of the present invention involves measuring cholesterol in lipoprotein using, as reagents, a cholesterol esterase and a cholesterol oxidase or cholesterol dehydrogenase, and a surfactant having a benzenesulfonic structure in the molecule or a polyacrylic acid-based polymeric surfactant, by subjecting a test sample containing lipoproteins to these reagents, and measuring the hydrogen peroxide or reduced coenzyme generated by the enzymatic reaction.

The enzymatic reaction of the present invention is carried out in an aqueous medium, and in particular, is preferably carried out in a buffer solution.
Examples of the buffering agent used in the buffer solution include tris(hydroxymethyl)aminomethane, phosphoric acid buffering agent, boric acid buffering agent, Good' s buffers, and the like. As for Good's buffers, there may be mentioned N-(2-acetamido)-2-aminoethanesulfonic acid (ACES), N-(2-acetamido)iminodiacetic acid (ADA), N,N-bis(2-hydroxyethyl)-2-aminoethanesulfonic acid (BES), 3-[N,N-bis(2-hydroxyethyl)amino]-2-hydroxypropanesulfonic acid (DIPSO), N-(2-hydroxyethyl)piperazine-N'-2-ethanesulfonic acid (HEPES), 2-(N-morpholino)ethanesulfonic acid (MES), 3-(N-morpholino)propanesulfonic acid (hereinafter, referred to as MOPS), 3-(N-morpholino)-2-hydroxypropanesuifonic acid (MOPSO), piperazine-N,N'-bis(2-ethanesulfonic acid) (PIPES), piperazine-N,N'-bis(2-hydroxypropane-3-sulfonic acid) (POPSO), and the like.

The pH value of the buffer solution is from 4 to 10, and preferably from 5 to 9. The use concentration of the buffer solution is preferably from 5 to 500 mM, more preferably from 10 to 200 mM, and particularly preferably 20 to 100 mM.

The cholesterol esterase is not particularly limited as long as it is an enzyme having an ability to hydrolyze a cholesterol ester, and may be exemplified by a cholesterol esterase, a lipoprotein lipase or the like, derived from a microorganism or an animal. A large number of this enzyme family is known to frequently have the esterase activity as well as a lipoprotein lipase activity, and in the present invention, it is preferable to use at least one cholesterol esterase having a stronger lipoprotein lipase activity compared to the cholesterol esterase activity.

Specifically, it is preferable to use at least one or more cholesterol esterase having an activity ratio between the activity as lipoprotein lipase and the activity as cholesterol esterase (lipoprotein lipase activity/cholesterol esterase activity) of 12 to 7000, preferably 20 to 1000, and more preferably 28 to 800. Byusing this enzyme, the selectivity of the enzyme to remnant-like lipoprotein can be enhanced. In addition, the lipoprotein lipase activity represents the activity unit obtained through calculation by a lipoprotein lipase activity assay using an olive oil emulsion (Horiuchi Y. et al., J. Biochem. 1976; 80:367-370), while the cholesterol esterase activity represents the activity unit obtained through calculation by a CEN activity assay using calf serum (Kameno Y. et al., Jap. J. Clin. Path. 1976; 24:650).

Examples of the cholesterol esterase having such activity ratio include LP (manufactured by Asahi Kasei Pharma Corp., activity ratio 460 to 800), LPBP (manufactured by Asahi Kasei Pharma Corp., activity ratio 5600 to 6900), CEN (manufactured by Asahi Kasei Pharma Corp., activity ratio 13.0 to 16.0), CE (manufactured by Amano Enzyme Inc. , activity ratio 13.0 to 16.0), COE311 (manufactured by Toyobo Co., Ltd., activity ratio 28.0 to 35.0,), and enzymes having activity ratios that are equivalent to the ratios of the above-mentioned enzymes. It is more preferable to use them in combination. Specifically, a combination of CEN and LP, a combination of COE311 and LP, and the like may be mentioned.

The cholesterol oxidase is not particularly limited so long as it is an enzyme having an ability to oxidize cholesterol and generate hydrogen peroxide, and examples thereof include cholesterol oxidases derived from microorganisms or animals.

The cholesterol dehydrogenase is not particularly limited so long as it is an enzyme having an ability to oxidize cholesterol and reducing an oxidized coenzyme, and examples thereof include cholesterol dehydrogenases derived from microorganisms or animals.

Furthermore, these enzymes may be enzymes chemically modified with a group mainly composed of polyethylene glycol, a water-soluble oligosaccharide residue, a sulfopropyl group or the like, so as to further enhance the specificity and stability of the enzyme, or may also be improved enzymes having equivalent activity, obtained by genetic manipulation.

The amounts of use of the cholesterol esterase, cholesterol oxidase and cholesterol dehydrogenase are each preferably 0.01 to 200 U/mL, and more preferably 0.1 to 100 U/mL, in the reaction solution.

The surfactant having a benzenesulfonic acid structure in the molecule or the polylacrylic acid-based polymeric surfactant may be a surfactant capable of improving the selectivity of the cholesterol esterase and the cholesterol oxidase or cholesterol dehydrogenase to the remnant-like lipoproteins, that is, a surfactant which enhances the action of the enzymes to the remnant-like lipoprotein, or relatively enhances the enzymatic response to the remnant-like lipoprotein by reducing the action of the enzymes to lipoproteins other than the remnant-like lipoprotein. Suitable examples of the surfactant having a benzenesulfonic acid structure in the molecule include alkylbenzensulfonic acids such as laurylbenzenesulfonic acid and dodecylbenzenesulfonic acid, polystyrene sulfonic acid, alkyl diphenyl ether disulfonic acid, and salts thereof, and examples of the salts include sodium salts, potassium salts, calcium salts, magnesium salts, ammonium salts and the like. Specifically, for example, Newrex R (sodium alkylbenzenesulfonate; Nippon Oil & Fats Co., Ltd.), Neopelex No. 6, Neopelex No. 25 (sodium dodecylbezenesulfonate; Kao Corp.), Pelex SS-L (sodium alkyl diphenyl ether disulfonate; Kao Corp.), calcium polystyrene sulfonate (Sanwa Chemical Co., Ltd.) may be suitably mentioned. Among them, it is particularly preferable to use Newrex R and Pelex SS-L from the viewpoint of increasing the selectivity of the enzymatic reaction.

The polyacrylic acid-based polymeric surfactant may be exemplified by polyacrylic acid salts, crosslinked polyacrylic acid salts, polyacrylic acid salt copolymers, or the like, and examples of the salts include sodium salts, potassium salts and the like. Sodium salts are particularly preferred. Specifically, for example, Jurymer AC-103 (sodium polyacrylate; Nihon Junyaku Co. , Ltd.), Jurymer AC-20N (sodium polyacrylate copolymer; Nihon JunyakuCo. , Ltd. ) , Rheogic 252L, Rheogic 306L (crosslinkedsodium polyacrylate; Nihon Junyaku Co., Ltd.) and the like maybe mentioned, and among these, Jurymer AC-103 is preferred.

The concentration to use such surfactant is not particularly limited, but is preferably 0.001 to 10%, more preferably 0.01 to 5%, and particularly preferably 0.05 to 1%.

With regard to the measurement method of the present invention, it is preferable to additionally use an anti-apoB-100 antibody which is reactive to the apolipoprotein B-100 contained in normal lipoprotein, and not reactive to denatured apolipoprotein B-100. Then, enzymatic reactions involving lipoproteins other than the remnant-like lipoprotein can be inhibited, and thus the enzymatic reaction involving the remnant-like lipoprotein can be relatively enhanced. Such anti-apoB-100 antibody may be any of a polyclonal antibody and a monoclonal antibody, but for example, an anti-human apoB-100 mouse monoclonal antibody (JI-H, JIMRO Co., Ltd.) is suitable.

The concentration to use such antibody is not particularly limited, but is preferably 0.01 to 10 mg/mL, and more preferably 0.1 to 5 mg/mL.
Furthermore, the anti-apoB-100 polyclonal antibody can be produced as follows. That is, serum LDL of a normal person, which is to be used as an immunogen, can be purified by a standard method and used, but for example, can be suitably prepared by an ultracentrifugation method (New Lectures on Biochemical Experiments 3 "Membrane lipids and plasma lipoproteins," p. 597 to 612). Subsequently, an animal other than human being is immunized once or multiple times with the purified LDL, and blood is collected at a time point where the antibody titer in the serum has increased, so that the antibody can be obtained in the form of antiserum. Also, when an antibody reactive to the remnant-like lipoprotein is present in the antiserum, the remnant-like lipoprotein is added as necessary to absorb the lipoprotein, and thereby the antiserum can be used in the measurement.

The production procedure of the anti-apoB-100 monoclonal antibody is as follows. First, the above-described purified LDL derived from the serum of a healthy person is suspended in Freund' s adjuvant or the like as an antigen, and is used to immunize mice several times. After completion of the immunization, extracted spleen cells are fused with mouse myeloma cells (for example, NS-1 cells, etc.) to produce a hybridoma, and thus a clone is obtained (JP-B-4-53516). As for the screening of a clone producing a target antibody, the affinity to the above-described purified LDL of healthy person and the affinity to a purified LDL derived from a hyperlipidemic patient are compared, and the hybridoma which strongly binds to the purified LDL of healthy person is selected, thereby obtaining the desired clone. The monoclonal antibody JI-H used in the present Examples was also produced according to this procedure.

As for the animal for immunization, an animal other than mouse, for example, rat or the like, can also be used according to necessity. In this case, Y3, Ag123 cells and the like can be suitably used as the myeloma cell.

In the measurement method of the present invention, when a cholesterol esterase and a cholesterol oxidase are used, hydrogen peroxide is generated from oxygen by the reaction of cholesterol. The generated hydrogen peroxide can be quantified, for example, using 4-aminoantipyrine and a phenol, 4-aminoantipyrine and Trinder reagent, or a high sensitivity chromogen, in the presence of a peroxidase.

Examples of the phenol include phenol, 4-chlorophenol, m-cresol, 3-hydroxy-2,4,6-triiodobenzoic acid (HTIB) and the like.

Examples of the Trinder reagent (Dojindo Laboratories, General Catalogue 19th ed., 2002) include anilines such as N-sulfopropylaniline, N-ethyl-N-(2-hydroxy-3-sulfopropyl)-m-toluidine (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-sulfopropyl-m-toluidine (TOPS), N-(2-hydroxy-3-sulfopropyl)-3,5-dimethoxyaniline (HDAOS), N,N-dimethyl-m-toluidine, N,N-disulfopropyl-3,5-dimethoxyaniline, N-ethyl-N-sulfopropyl-m-anisidine, N-ethyl-N-sulfopropylaniline, N-ethyl-N-sulfopropyl-3,5-dimethoxyaniline, N-sulfopropyl-3,5-dimethoxyaniline, N-ethyl-N-sulfopropyl-3,5-dimethylaniline, N-ethyl-N-(2-hydroxy-3-sulfopropyl)-m-anisidine, N-ethyl-N-(2-hydroxy-3-sulfopropyl)aniline, N-ethyl-N-(2-hydroxy-3-sulfopropyl)-3,5-dimethoxyaniline, N-ethyl-m-3-sulfopropyl-3-methoxyaniline (APPS), N-ethyl-N-3-sulfopropylaniline (ALPS) and N-ethyl-N-2-hydroxy-3-sulfoproxyl-3-methoxyaniline; N-ethyl-N-(3-methylphenyl)-N'-succinylethylenediamine (EMSE), N-ethyl-N-(3-methylphenyl)-N'-acetylethylenediamine, and the like.

As the high sensitivity chromogen, 10-(N-methylcarbamoyl)-3,7-bis(dimethylamino)phenothiazine (MCDP) disclosed in JP-B-60-33479, bis[3-bis(4-chlorophenyl)methyl-4-dimethylaminophenyl]amine (BCMA) disclosed in JP-B-4-27839, the compound disclosed in JP-A-62-296, and the like may be mentioned.

The concentration of these chromogens is preferably 0.01 to 10 mM. Also, when a cholesterol esterase and a cholesterol dehydrogenase are used, a reduced coenzyme NAD (P) H is generated from an oxidized coenzyme NAD (P) by the reaction of cholesterol. NAD(P)H can be quantified by measuring the absorbance of the reaction solution at 300 to 500 nm, preferably 330 to 400 nm, and particularly preferably at about 340 nm. In addition, quantification of NAD(P)H can also be performed by adding diaphorase and a tetrazolium salt to produce a formazane dye, and colorimetrically measuring the formazane dye.
The reaction is performed at 10 to 50°C, preferably 30 to 40°C, and usually at 37°C, for 1 to 30 minutes, and preferably 2 to 10 minutes.

The measurement method of the present invention comprises subjecting a test sample to the action of the above-described reagents, but the method of addition or order of addition of the sample, and the like are not particularly limited, as long as the order of action is maintained such that the test sample is reacted with the surfactant or with the surfactant and apolipoprotein B-100 contained in a normal lipoprotein, then with an anti-apoB-100 antibody which is not reactive to denatured apolipoprotein B-100, and then with a cholesterol esterase and a cholesterol oxidase or cholesterol dehydrogenase. Furthermore, since the reaction is performed in an aqueous medium, it is preferable to follow the procedure of preparing the test sample by adding to an aqueous medium composed of a buffer solution or the like, while stirring at 25°C to 40°C, then adding the reagents of the present invention, allowing the sample and the reagents to react for 1 to 10 minutes, and making measurements.

Therefore, the constitution of the reagent of the present invention may be any of a constitution of separately providing a reagent containing a surfactant, a reagent containing a surfactant and an anti-apoB-100 antibody which is reactive to apolipoprotein B-100 contained in normal lipoproteins, but is not reactive to denatured apolipoprotein B-100, and a reagent containing a cholesterol esterase and a cholesterol oxidase or cholesterol dehydrogenase, and a constitution of combining these reagents into a single preparation. In the case of providing the reagents separately, they can be used by adding one by one, or by adding all at once.
Furthermore, each of the reagents may be in a liquid form, or may also be in a solid form such as tablet or powder.

The reagents for measurement can contain, if necessary, various salts for solubilizing proteins in the biological sample, such as globulins, a lipoprotein aggregating agent, other enzymes and the like.
Examples of such salts include sodium chloride, sodium sulfate, potassium chloride, potassium sulfate, magnesium chloride, magnesium sulfate, magnesium acetate, lithium chloride, lithium sulfate, ammonium chloride, ammonium sulfate, magnesium nitrate, calcium nitrate, and the like, and the salts are used in the range of 0 to 100 mM.

Examples of the lipoprotein aggregating agent include phosphotungstic acid salts; polyanions such as dextran sulfuric acid salts and heparin; and salts of divalent metals such as magnesium, calcium and cobalt. Examples of the other enzymes include ascorbic acid oxidase and the like.
Additionally, the test sample that is applicable to the present invention is not particularly limited, and specifically, whole blood or blood fractions such as plasma and serum can be used as the biological sample.

### EXAMPLES

Hereinafter, the present invention will be described in more detail by way of Examples.

### Example 1

### (1) Preparation of sample

Blood serum component was fractionated into a remnant-like lipoprotein fraction, an LDL fraction and an HDL fraction, and each of the lipoprotein fractions was measured. The method of protein fractionation will be described below.

An anti-apoB-100 antibody-bound affinity column and an anti-apoA-1 antibody-bound affinity column were respectively prepared, blood serum was added thereto, and bound fractions were recovered and assigned as LDL and HDL, respectively. Also, blood serum was added to the above-mentioned two columns, and a pass-through fraction was recovered and assigned as remnant-like lipoprotein fraction (RLP). The lipoproteins were each confirmed by an HPLC analysis. Also, the cholesterol concentration in each of the lipoproteins was adjusted to 5 mg/dl using a reagent kit for total cholesterol measurement.

### (2) Preparation of reagents for measurement

The reagents for measuring cholesterol in the remnant-like lipoprotein as shown below were prepared.

**[Table 1]**

| Reagent 1 |
|---|
| Good's buffer pH 6.8 (PIPES, manufactured by Dojindo Laboratories): 50 mM |
| HDAOS (manufactured by Dojindo Laboratories): 1 mM |
| Surfactant (see Table 1; invented products 1 and 2, comparative products 2 to 8) |
| |

| Reagent 2 |
|---|
| Good's buffer pH 6.8 (BES, manufactured by Dojindo Laboratories): 50 mM |
| 4-Aminoantipyrine (manufactured by Kanto Chemical Co., Inc.): 3 mM |
| Peroxidase (manufactured by Amano Enzyme, Inc.): 10 units/mL |
| Cholesterol esterase (COE311: activity ratio 31.8, manufactured by Toyobo Co., Ltd.): 15 units/mL |
| Cholesterol oxidase (manufactured by Amano Enzyme, Inc.): 6 units/mL |

### (3) Measurement method and results

210 µL of reagent 1 was placed in a spectrophotometric cell, and 20 µL of the sample was added thereto. The mixture was stirred and heated to 37°C. Then, after 344 seconds, 70 µL of reagent 2 was added to the mixture and stirred, and measurement was performed at a main wavelength of 572 nm and a subwavelength of 748 nm, up to 667 seconds after.

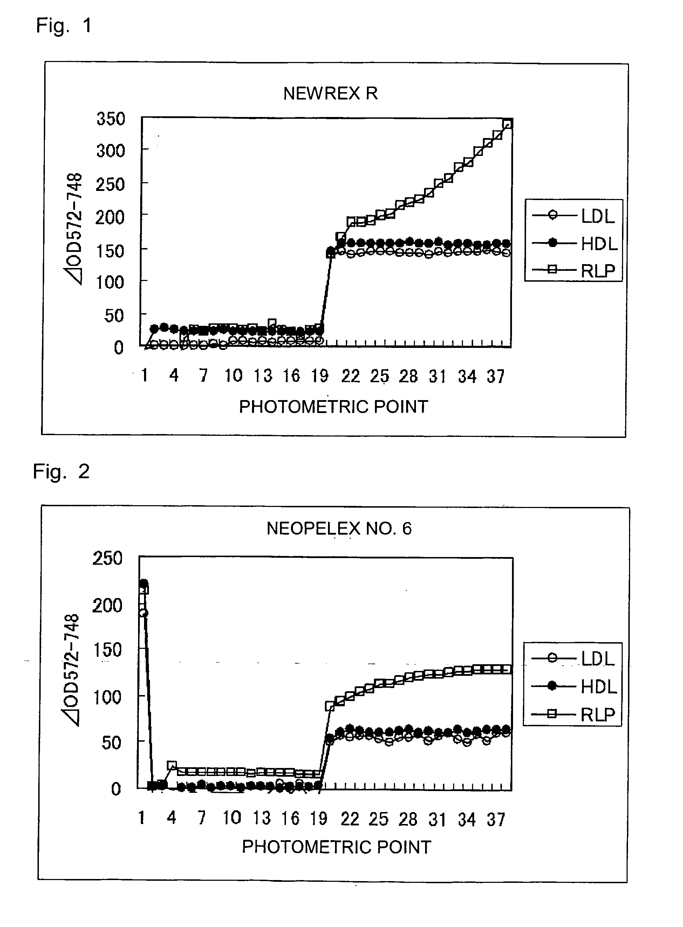

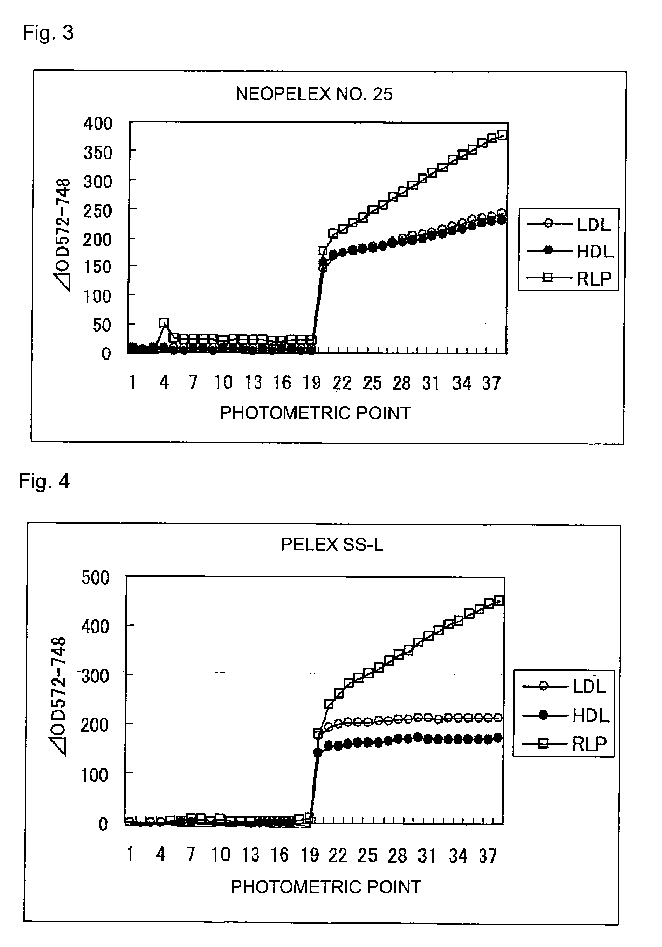

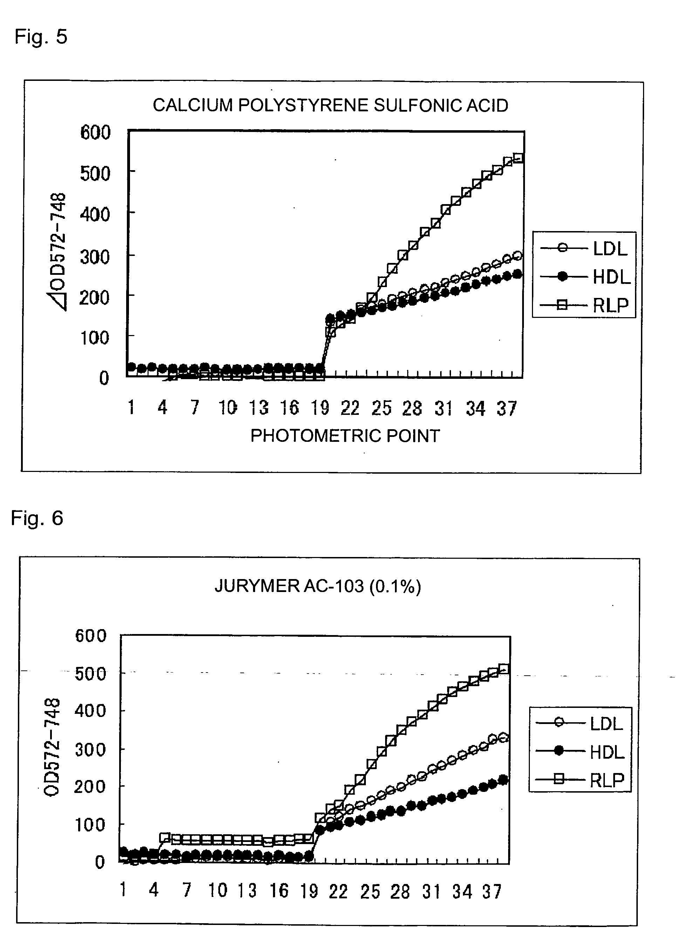

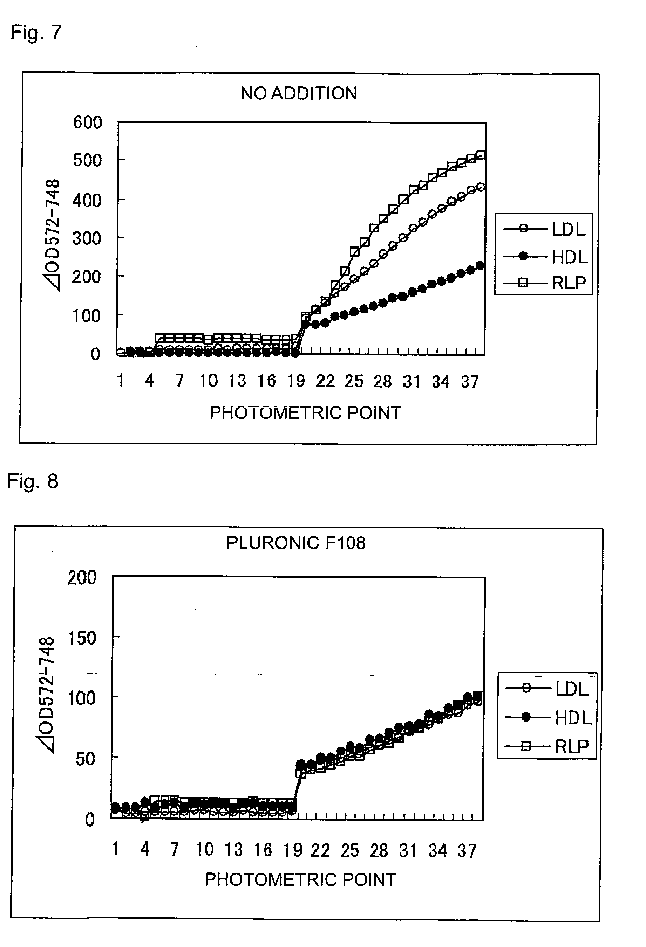

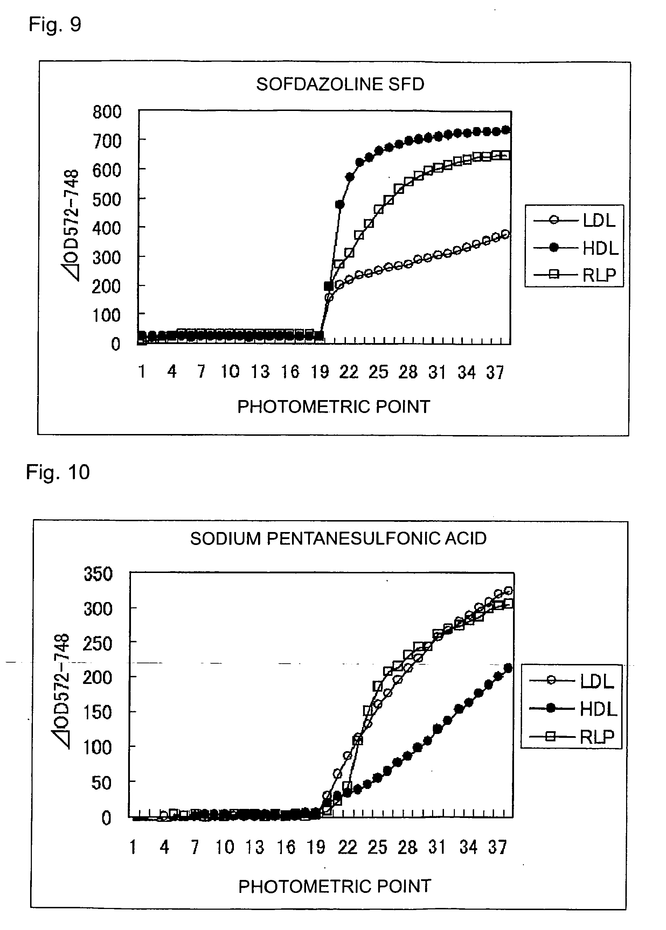

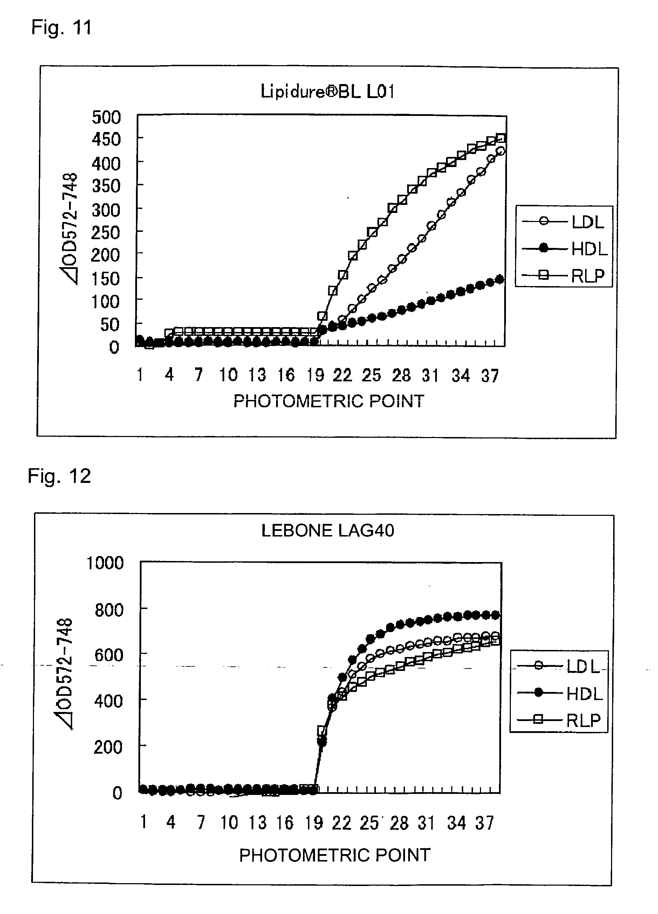

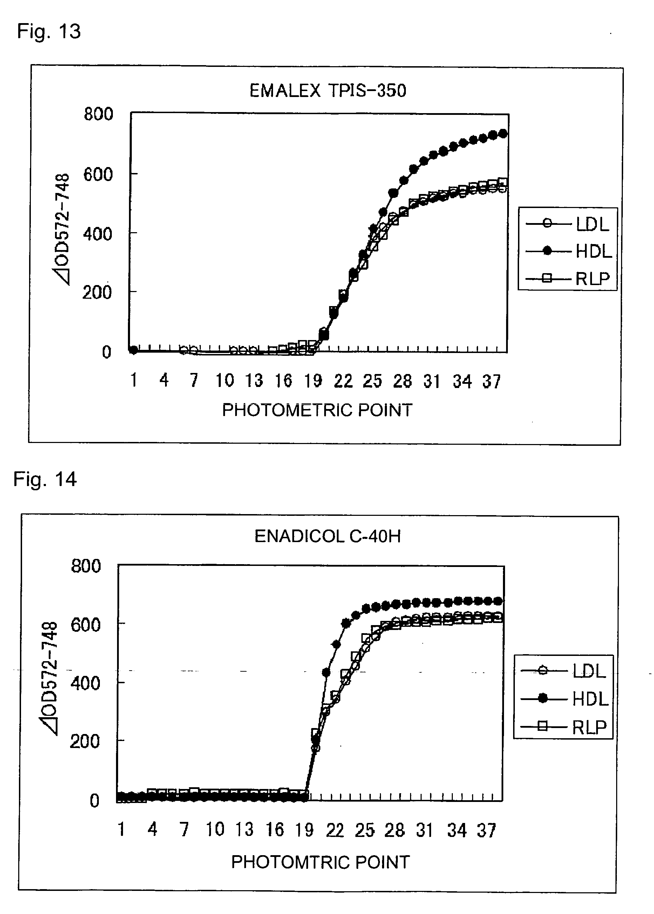

The amount of change in the absorbance upon the addition of reagent 2, that is, from after 344 seconds (photometric point 20) to after 667 seconds (photometric point 38) was determined, and the relative ratios in the case where the amount of change in the absorbance of RLP was taken as 100, were calculated for RLP, HDL and LDL. The results are presented in Table 2 and Figs. 1 to 14.

**[Table 2]**

| Reagent | Compound | Manufactur er | Cone ntrat ion | Class | Product name | Relative ratio | | |
|---|---|---|---|---|---|---|---|---|
| | | | | | | LDL | HDL | RLP |
| The invented product 1 | Sodium alkylbenzenesulfonate | Nippon Oil & Fats Co., Ltd. | 0.1% | Anionic surfactant | Newrex R | 3 | 8 | 100 |
| The invented product 2 | Sodium alkylbenzenesulfonate | Kao Corp. | 0.1% | Anionic surfactant | Neopelex No. 6 | 33 | 24 | 100 |
| The invented product 3 | Sodium alkylbenzenesulfonate | Kao Corp. | 0.1% | Anionic surfactant | Neopelex no. 25 | 53 | 35 | 100 |
| The invented product 4 | Sodium alkyl diphenyl ether disulfonate | Kao Corp. | 0.1% | Anionic surfactant | Pelex SS-L | 14 | 11 | 100 |
| The invented product 5 | Calcium polystyrene sulfonate | Sanwa Chemical Co., Ltd. | 0.1% | Anionic surfactant | Polystyrene sulfonic acid Ca | 43 | 29 | 100 |
| The invented product 6 | Sodium polyacrylate | Nihon Junyaku Co., Ltd. | 0.1% | Polymeric surfactant | Jurymer AC-103 | 65 | 35 | 100 |
| Comparati ve product 1 | No addition | - | - | - | - | 93 | 35 | 100 |
| Comparati ve product 2 | Polyoxyethylene-polyo xypropylene block polymer | AsahiDenka Co., Ltd. | 0.1% | Nonionic surfactant | Pluronic F108 | 17 | 285 | 100 |
| Comparati ve product 3 | Hydroxyethyl ethylenediamine sodium | Kawaken Fine Chemicals Co., Ltd. | 0.1% | Amphoteric surfactant | Sofdazoline SFD | 47 | 102 | 100 |
| Comparati ve product 4 | Sodium pentanesulfonate | Avocado Research Chemicals Ltd. | 0.1% | Anionic surfactant | Sodium pentanesulf onate | 80 | 40 | 100 |
| Comparati ve product 5 | 2-Methacryloyloxyethy 1 phosphorylcholine copolymer | Nippon Oil & Fats Co., Ltd. | 0.1% | Phospholipid polymer | Lipidure^{□} BL L01 | 97 | 26 | 100 |
| Comparati ve product 6 | Alkyldiaminoethylglyc ine hydrochloride | Sanyo Chemical Industries , Ltd. | 0.1% | Amphoteric surfactant | Lebon LAG40 | 104 | 124 | 100 |
| Comparati ve product 7 | Polyoxyethylene triisostearic acid trimethylolpropane | Nihon Emulsion Co., Ltd. | 0.1% | Nonionic surfactant | EMALEX TRIS-350 | 128 | 142 | 100 |
| Comparati ve product 8 | Imidazolinium betaine | Lion Corp. | 0.1% | Amphoteric surfactant | Enadicol C-40H | 123 | 118 | 100 |

As seen from Table 2 and Figs. 1 to 14, the invented products 1 to 6 of the present invention inhibited all of the enzymatic reactions involving LDL and HDL, and had the selectivity of enzymatic action to RLP remarkably increased, as compared with the comparative products 1 to 8.

### Example 2

Subsequently, the amount of RLP-cholesterol in human blood serum was measured according to the measurement method of the present invention, using an automatic analyzer TBA-20R (Toshiba Corp.). In addition, Newrex R (invented product) was used as the surfactant.
Sample: human blood serum 106 specimens

[Table 3]
Reagent 1
Good's buffer pH 6.8 (PIPES, manufactured by Dojindo Laboratories): 50 mM
HDAOS (manufactured by Dojindo Laboratories): 1 mM
Newrex R (manufactured by Nippon Oil & Fats Co., Ltd. ) : 0.01%
Reagent 2
Good's buffer pH 6.8 (BES, manufactured by Dojindo Laboratories): 50 mM
4-Aminoantipyrine (manufactured by Kanto Chemical Co., Inc.): 3 mM
Peroxidase (manufactured by Amano Enzyme, Inc.): 10 units/mL
Cholesterol esterase (COE311: activity ratio 31.8, manufactured by Toyobo Co., Ltd.): 15 units/mL
Cholesterol oxidase (manufactured by Amano Enzyme, Inc.): 6 units/mL

The measurement was performed by placing 210 µL of reagent 1 in a spectrophotometric cell, adding 5 µL of blood serum with stirring, and heating the mixture to 37°C. Then, after344 seconds, 70 µL of reagent 2, pre-heated to 37°C, was added thereto while stirring, and measurement was performed at a main wavelength of 572 nm and a subwavelength of 748 nm, up to 667 seconds after.

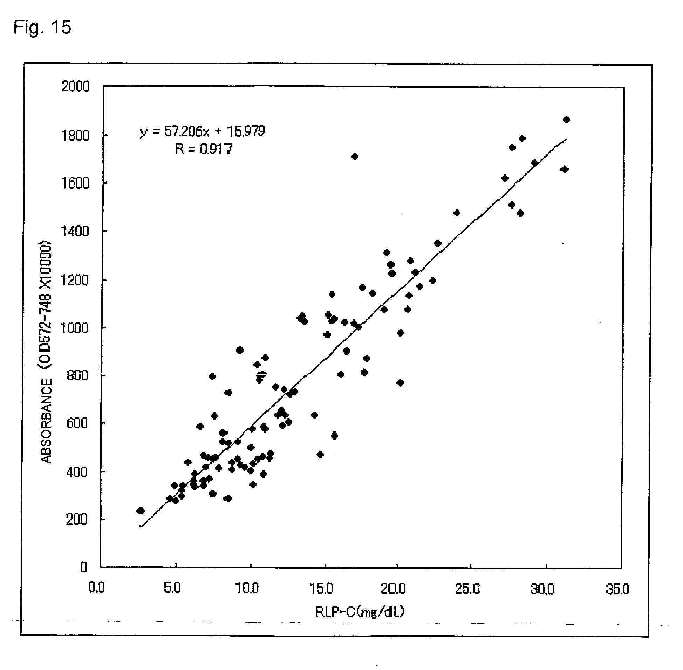

The absorbance values at photometric points 34 to 38 (595 to 667 seconds) (blank: photometric points 15 to 19 (251 to 328 seconds)) were determined, and the correlation between the above-obtained values and the values obtained with a commercially available RLP-cholesterol measurement kit (manufactured by JIMRO Co., Ltd.) was determined (Fig. 15). As a result, as shown in Fig. 15, a high correlation was observed between the values determined by the measurement method of the present invention and the values obtained with a commercially available kit.

### Example 3

The amount of RLP-cholesterol in human blood serum was measured according to the measurement method of the present invention, using an automatic analyzer 7080 (Hitachi, Ltd.). In addition, PelexSS-L (invented product 4) was used as the surfactant, and COE311 (activity 31.8) and LP (activity ratio 512) were used as the cholesterol esterase.
Sample: human blood serum 30 specimens

[Table 4]
Reagent 1
Good's buffer pH 6.5 (PIPES, manufactured by Dojindo Laboratories): 50 mM
HDAOS (manufactured by Dojindo Laboratories): 1 mM
Pelex SS-L: 0.02%
Reagent 2
Good's buffer pH 6.5 (PIPES, manufactured by Dojindo Laboratories): 50 mM
4-Aminoantipyrine (manufactured by Kanto Chemical Co., Inc.): 3 mM
Peroxidase (manufactured by Amano Enzyme, Inc.): 10 units/mL
Cholesterol esterase (COE311: activity ratio 31.8, manufactured by Toyobo Co., Ltd.): 10 units/mL
Cholesterol esterase (LP: activity ratio 512, manufactured by Asahi Kasei Pharma Corp.): 20 units/mL

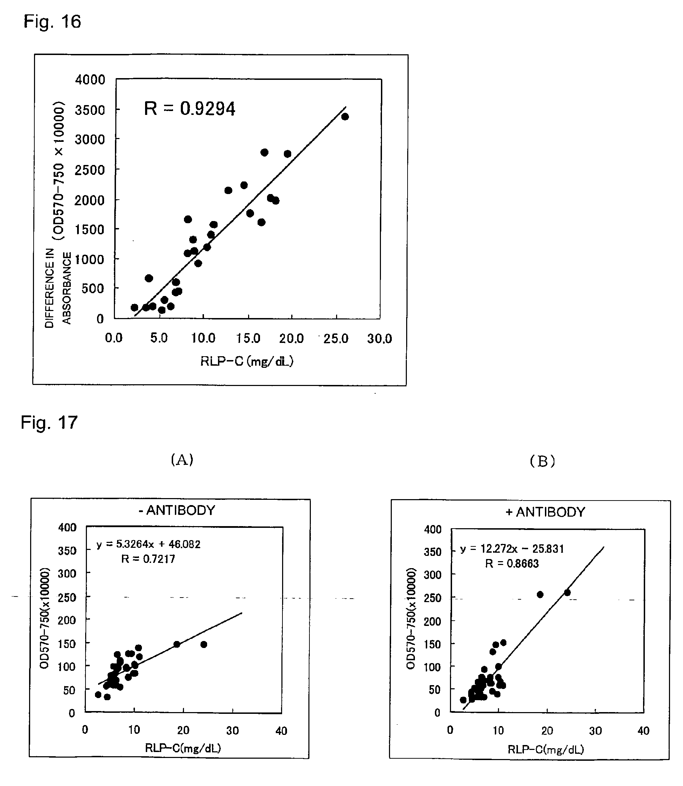

The measurement was performed by placing 5 µL of blood serum in a spectrophotometric cell, adding 210 µL of reagent 1 with stirring, and heating the mixture to 37°C. Then, after 320 seconds, 70 µL of reagent 2 was added thereto while stirring, and measurement was performed at a main wavelength of 570 nm and a subwavelength of 750 nm, up to 980 seconds after.
The differences in absorbance values at photometric point 33 (660 seconds) and at photometric point 17 (340 seconds) were determined, and the correlation between these values and the values obtained with a commercially available RLP-cholesterol measurement kit (manufactured by JIMRO Co., Ltd.) was determined (Fig. 16). As a result, as shown in Fig. 16, a high correlation (R=0.9294) was observed between the values determined by the measurement method of the present invention and the values obtained with a commercially available kit.

### Example 4

The amount of RLP-cholesterol in blood serum was measured according to the measurement method of the present invention, using an automatic analyzer 7080 (Hitachi, Ltd.).
Sample: human serum 27 specimens

[Table 5]
Reagent 1
PIPES (pH 6.8) (manufactured by Dojindo Laboratories): 50 mM
Cholesterol oxidase (manufactured by Amano Enzyme, Inc.) :2 U/mL
Pelex SS-L (manufactured by Kao Corp.): 0.05%
HDAOS (manufactured by Dojindo Laboratories): 1 mM
Anti-apoB-100monoclonal antibody (JI-H, manufactured by JIMRO Co., Ltd.): 1 mg/mL
Reagent 2
BES (pH 6.8) (manufactured by Dojindo Laboratories): 50 mM
Cholesterol esterase (manufactured by Toyobo Co., Ltd.): 15 U/mL
Peroxidase (manufactured by Amano Enzyme, Inc.): 10 U/mL
4-Aminoantipyrine (manufactured by Kanto Chemical Co., Inc.): 3 mM
Bovine serum albumin (manufactured by Sigma-Aldrich Japan KK) : 0.1%

### Measurement conditions and method

The measurement parameters were set as follows, and measurement was performed.
Measurement method: Rate method test read timing [22] to [25]
Amount of sample: 5 µL
Amount of R1: 210 µL
Amount of R2: 70 µL
Measurement wavelength: Main wavelength 570 nm
Subwavelength: 750 nm
Measurement temperature: 37°C

210 µL of reagent 1 was added to 5 µL of the specimen with stirring, and the mixture was heated to 37°C. Then, after 5 minutes, 70 µL of reagent 2 was added with stirring, and measurement was performed at a main wavelength of 570 nm and a subwavelength of 750 nm. The amount of change in the absorbance per one minute after the addition of reagent 2 was determined, and the correlation between the above-obtained values and the values obtained with a commercially available RLP-cholesterol measurement kit (manufactured by JIMRO Co., Ltd.) was determined (Fig. 17). As a result, as shown in Fig. 17, a higher correlation between the values determined by the measurement method of the present invention and the values obtained with a commercially available kit, was observed in the case of adding the anti-apoB-100 antibody, as compared with the case of no antibody addition.

## Claims

1. A method for measuring cholesterol in remnant-like lipoprotein, the method comprising subjecting a test sample containing lipoproteins to a cholesterol esterase and a cholesterol oxidase or cholesterol dehydrogenase, and measuring the hydrogen peroxide or reduced coenzyme generated by the enzymatic reaction, wherein a surfactant having a benzenesulfonic acid structure in the molecule or a polyacrylic acid-based polymeric surfactant is used.

2. The method according to claim 1, further employing an anti-apoB-100 antibody which is reactive to apolipoprotein B-100 contained in normal lipoproteins and which is not reactive to denatured apolipoprotein B-100.

3. The method according to claim 1 or 2, wherein the test sample is subjected to the action of the surfactant, or to the surfactant and an anti-apoB-100 antibody which is reactive to apolipoprotein B-100 contained in normal lipoproteins and which is not reactive to denatured apolipoprotein B-100, and then is subjected to the action of the cholesterol esterase and the cholesterol oxidase or cholesterol dehydrogenase.

4. The method according to any one of claims 1 to 3, wherein alkylbenzenesulfonic acid, polystyrene sulfonic acid, alkyl diphenyl ether disulfonic acid, or a salt thereof, or a polyacrylic acid salt is used as the surfactant.

5. The method according to any one of claims 1 to 4, wherein the method comprises using at least one cholesterol esterase having an activity ratio between the activity as lipoprotein lipase and the activity as cholesterol esterase (lipoprotein lipase activity/cholesterol esterase activity) of 12 to 7000.

6. A reagent for measuring cholesterol in remnant-like lipoprotein, the reagent comprising a cholesterol esterase and a cholesterol oxidase or cholesterol dehydrogenase, and a surfactant having a benzenesulfonic acid structure in the molecule or a polyacrylic acid-based polymeric surfactant.

7. The reagent according to claim 6, further comprising an anti-apoB-100 antibody which is reactive to apolipoprotein B-100 contained in normal lipoproteins and which is not reactive to denatured apolipoprotein B-100.

8. The reagent according to claim 6 or 7, wherein the surfactant is alkylbenzenesulfonic acid, polystyrene sulfonic acid, alkyl diphenyl ether disulfonic acid, or a salt thereof, or a polyacrylic acid salt.

9. The reagent according to any one of claims 6 to 8, wherein the reagent comprises at least one cholesterol esterase having an activity ratio between the activity as lipoprotein lipase and the activity as cholesterol esterase (lipoprotein lipase activity/cholesterol esterase activity) of 12 to 7000.
